(19) Europäisches Patentamt
European Patent Office
Office européen des brevets

(11) **EP 3 852 675 B1**

(12) **EUROPÄISCHE PATENTSCHRIFT**

(45) Veröffentlichungstag und Bekanntmachung des Hinweises auf die Patenterteilung:
**01.02.2023 Patentblatt 2023/05**

(21) Anmeldenummer: **19768839.3**

(22) Anmeldetag: **18.09.2019**

(51) Internationale Patentklassifikation (IPC):
***A61C 7/08*** *(2006.01)*

(52) Gemeinsame Patentklassifikation (CPC):
**A61C 7/08;** A61C 2201/00

(86) Internationale Anmeldenummer:
**PCT/EP2019/074959**

(87) Internationale Veröffentlichungsnummer:
**WO 2020/058311 (26.03.2020 Gazette 2020/13)**

(54) **3D-GEDRUCKTE KIEFERORTHOPÄDISCHE ZAHNSCHIENE AUS VERNETZTEN POLYMEREN**

3D-PRINTED ORTHODONTIC DENTAL SPLINT MADE OF CROSS-LINKED POLYMERS

APPAREIL ORTHODONTIQUE D’ORTHOPÉDIE MAXILLAIRE IMPRIMÉE EN 3D À PARTIR DE POLYMÈRES RÉTICULÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.09.2018 EP 18195715**

(43) Veröffentlichungstag der Anmeldung:
**28.07.2021 Patentblatt 2021/30**

(73) Patentinhaber: **Covestro Intellectual Property GmbH & Co. KG**
**51373 Leverkusen (DE)**

(72) Erfinder:
- **ACHTEN, Dirk**
  **51375 Leverkusen (DE)**
- **WAGNER, Roland**
  **51375 Leverkusen (DE)**
- **TOMCZYK, Christoph**
  **51379 Leverkusen (DE)**
- **BÜSGEN, Thomas**
  **51377 Leverkusen (DE)**

(74) Vertreter: **Davepon, Björn**
**Patentanwaltskanzlei Davepon**
**Schloss Dyck**
**41363 Jüchen (DE)**

(56) Entgegenhaltungen:
**WO-A1-2018/005501**
**WO-A1-2018/119026**
**US-A1- 2009 148 813**
**US-A1- 2017 007 386**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 852 675 B1

**Beschreibung**

Die vorliegende Erfindung betrifft eine kieferorthopädische Zahnschiene aus einem vernetzten Polymer, wobei das vernetzte Polymer eine Glasübergangstemperatur $T_g$, bestimmt mittels dynamisch-mechanischer Analyse bei einer Frequenz von 1/s als Peak tan $\delta$, von $\geq$ 25 °C und $\leq$ 60 °C, ein Elastizitätsmodul, bestimmt mittels dynamisch-mechanischer Analyse als Speichermodul E' bei einer Frequenz von 1/s bei 35 °C, von $\geq$ 500 MPa und $\leq$ 4000 MPa und einen Verlustfaktor tan $\delta$, bestimmt mittels dynamisch-mechanischer Analyse bei einer Frequenz von 1/s bei 35 °C, von $\geq$ 0,08 aufweist. Die Erfindung betrifft weiterhin ein Verfahren zur Herstellung ebensolcher Zahnschienen.

Die Korrektur von Zahnfehlstellungen kann neben ästhetischen auch auf gesundheitlichen Gründen beruhen. Eine korrekte Zahnstellung kann für eine schmerzfreie, funktionale Ausrichtung zwischen Ober- und Unterkiefer wichtig sein und zudem kann das Auftreten weiterer Zahnkrankheiten, wie beispielsweise Parodontose oder Karies, durch falsch ausgerichtete Zähne und damit einhergehende, ungünstigere Zahnreinigungsmöglichkeiten gefördert werden. Aus diesen Gründen wurden schon sehr früh unterschiedlichste Geräte eingesetzt, um die Stellung einzelner oder sämtlicher Zähne des Zahnapparates eines Anwenders in eine natürliche und ästhetische Form zu bringen. Angefangen von einfachen Holzspateln zur manuellen Korrektur einzelner Zähne über mehr oder minder aufwendige Kieferverdrahtungen bis hin zu Zahnspangen wurden viele Gerätschaften genutzt, um über mechanischen Druck die Zähne von einer ursprünglichen Fehl- hin zu einer "richtigen" Zielposition zu bewegen. Das Behandlungsergebnis ergab sich als Funktion aus Anwendungsdauer und ausgeübtem Druck, wobei letzterer sich aus der Kombination aus dem strukturellen Aufbau der Hilfsmittel und den verwendeten Materialien ergibt.

In den letzten Jahren haben sich neben verklebten Draht-Spangen, gegossene und auch über 3D-Druck individuell herstellbare kieferorthopädische Zahnschienen, sogenannte Aligner, zur Behandlung von Zahnreihen mit komplexen Fehlstellungen etabliert. Üblicherweise werden in diesen Verfahren "zukünftige" Zahnstellungen auf dem geplanten Behandlungspfad über einen rechnergesteuerten Prozess prognostiziert und diese angestrebten Zahnkonfigurationen über einen 3D-Druckprozess hergestellt. Die Produktion der Korrekturhilfen erfolgt dann aber über einen Tiefziehprozess spezieller Folien über das gedruckte 3D-Zahnmodell. Der eigentliche Aligner wird also in den allermeisten Fällen nicht direkt mittels 3D-Druck hergestellt. Im Stand der Technik finden sich jedoch auch verschiedene Herstellverfahren zur direkten Erzeugung von Zahnkorrekturschienen mittels 3D Druck.

So beschreibt beispielsweise die US 2016 025 6240 A1 die Herstellung von direktgedruckten Zahnkorrekturschienen nach verschiedenen 3D Druckverfahren, wobei das benutzte Material ein zugelastisches Verhalten zeigen soll. Als gewünschte Materialeigenschaften werden hierbei Materialeigenschaften genannt, wie sie aus der Literatur zu folienbasierten Zahnkorrekturschienen bekannt sind. Beispiele für Materialien, die diese Eigenschaften in 3D gedruckter Form erfüllen werden nicht genannt.

Die US 2013 0095 446 A1 beschreibt die Herstellung von Zahnkorrekturschienen nach einem 3D Druck Verfahren und listet kommerziell verfügbare Verfahren und eine willkürliche Liste von für die Verfahren geeigneten kommerziellen 3D verdruckbaren bio-kompatiblen Materialien auf, die gegebenenfalls zur Herstellung von Zahnkorrekturschienen geeignet sein könnten.

Die US 2013 0122 448 A1 beschreibt die Herstellung einer Zahnkorrekturschiene über den 3D Druck einer Negativform, die dann mit einem flüssigen Material als positiv Form ausgefüllt wird. Die gehärtete Positivform dient dann als Zahnkorrekturschiene.

U.S. Pat. No. 5,975,893 beschreibt die Herstellung tiefgezogener transparenter Zahnkorrekturschienen, wobei das Gebiss gescannt und verschiedene Korrekturpositionen errechnet werden. Davon werden dann Positivmodelle der zu korrigierenden Zähne und des Gebisses mittels eines 3D Druckers gedruckt. Um dieses gedruckte Modell herum wird eine Folie tiefgezogen und als transparente Zahnschiene aufgearbeitet. Bei der Folie handelt es sich um ein oder mehrere hochschmelzende hochmodulige Thermoplasten.

Das Dokument WO 2018/005501 A1 offenbart ein Verfahren zum Aufbauen eines dreidimensionalen Objekts unter Verwendung einer druckbaren Zusammensetzung, die hochviskose polymere Komponenten enthält.

Trotz der schon vorhandenen Alternativen im Stand der Technik existiert weiterhin Bedarf an geeigneten 3D verdruckbaren Materialien, welche sich zum Einsatz als kieferorthopädische Zahnschienen eignen und welche ein genau auf die Umgebungsbedingungen der Anwendung adaptiertes Eigenschaftsprofil aufweisen.

Eine Aufgabe der vorliegenden Erfindung ist es somit, mindestens einen Nachteil des Standes der Technik wenigstens zu einem Teil zu überwinden und Materialien für additive Fertigungsverfahren bereitzustellen, welche eine hohe Auflösung in der Herstellung ermöglichen, eine ausgezeichnete Biokompatibilität aufweisen und unter den physikalischen Bedingungen im Mundraum verbesserte Anwendungseigenschaften aufweisen. Des Weiteren ist eine Aufgabe der Erfindung, diese Gegenstände kosteneffizient und ressourcenschonend bereitstellen zu können.

Erfindungsgemäß gelöst wird die Aufgabe durch eine Zahnschiene gemäß Anspruch 1 und ein Verfahren gemäß Anspruch 9. Vorteilhafte Weiterbildungen sind in den Unteransprüchen angegeben. Sie können beliebig kombiniert werden, sofern sich aus dem Kontext nicht eindeutig das Gegenteil ergibt.

Vorgeschlagen wird eine kieferorthopädische Zahnschiene, wobei die Zahnschiene ein vernetztes Polymer

umfasst oder aus einem solchen besteht, wobei das vernetzte Polymer eine Glasübergangstemperatur $T_g$, bestimmt mittels dynamisch-mechanischer Analyse bei einer Frequenz von 1/s DMA als Peak tan δ, von ≥ 25 °C und ≤ 60 °C, ein Elastizitätsmodul, bestimmt mittels dynamisch-mechanischer Analyse als Speichermodul E' bei einer Frequenz von 1/s bei 35 °C, von ≥ 500 MPa und ≤ 4000 MPa und einen Verlustfaktor tan δ, bestimmt mittels dynamisch-mechanischer Analyse bei einer Frequenz von 1/s bei 35 °C, von ≥ 0,08 aufweist.

Das vernetzte Polymer ist ein Copolymer, welches Einheiten basierend auf einem ersten Monomer und einem zweiten Monomer enthält, wobei das erste Monomer ein (meth)acrylisches Monomer ist, dessen Homopolymer eine Glasübergangstemperatur, bestimmt mittels dynamisch-mechanischer Analyse bei einer Frequenz von 1/s als Peak tan δ, von ≤ 0 °C aufweist und das zweite Monomer ein (meth)acrylisches oder styrolisches Monomer ist, dessen Homopolymer eine Glasübergangstemperatur, bestimmt mittels dynamisch-mechanischer Analyse bei einer Frequenz von 1/s als Peak tan δ, von ≥ 60 °C aufweist und wobei Einheiten basierend auf dem ersten Monomers in einem Anteil von ≥ 5 bis ≤ 40 Gewichts-%, bezogen auf das Gesamtgewicht des vernetzten Polymers, vorliegen und Einheiten basierend auf dem zweiten Monomer in einem Anteil von ≥ 20 bis ≤ 80 Gewichts-%, bezogen auf das Gesamtgewicht des vernetzten Polymers, vorliegen. Die genannten Gewichts-%-Angaben addieren sich zu ≥ 25% bis ≤ 90 Gewichts-% der polymerisierbaren Zusammensetzung. Das erste Monomer kann als sogenanntes "low $T_g$ monomer" und das zweite als "high $T_g$ monomer" bezeichnet werden. Solche Monomere und die entsprechenden Glasübergangstemperaturen sind bekannt und kommerziell zugänglich. Beispiele für das erste Monomer sind n-Butylacrylat und sec-Butylacrylat, tert-Butylacrylat, n-Propylacrylat, 2-Propylacrylat, 2-Ethyhexylacryat und Ethylacrylat. Beispiele für das zweite Monomer sind Isobornylacrylat (IBOA) und Isobornylmethacrylat (IBOMA, Methylmethacrylat und in einer besonderen Ausführungsform, Styrol. Es können selbstverständlich auch mehrere verschiedene Vertreter der "low $T_g$"-Monomere und/oder der "high $T_g$"-Monomere dem Copolymer zugrunde liegen.

Überraschenderweise hat sich gezeigt, dass kieferorthopädische Zahnschienen mit oben genannten Parametern einen deutlich besseren Behandlungserfolg zeigen, als die üblicherweise verwendeten Zahnschienen. Ohne durch die Theorie gebunden zu sein kann dies daran liegen, dass das eingesetzte Zahnschienenmaterial ein auf die Anwendungstemperatur abgestimmtes Eigenschaftsprofil zeigt. Dies bedeutet, dass die eingesetzten Polymere temperaturabhängige Eigenschaften aufweisen und die hier beanspruchten bevorzugt vernetzten Polymere bei der Behandlungstemperatur im Mundraum eines Anwenders die erforderlichen mechanischen Eigenschaften aufbringen.

Dieses steht im Gegensatz zu dem im Stand der Technik beschriebenen Materialien, über deren Eignung im Wesentlichen anhand der Eigenschaften bei Raumtemperatur geurteilt wird. D.h. üblicherweise wird die Eignung eines Materials im Bereich zwischen 20 und 25 °C getestet und als Funktion der erhaltenen mechanischen Werte dieses Material entweder als geeignet oder ungeeignet eingestuft.

Bei diesem Ansatz wird allerdings außer Acht gelassen, dass die Einsatzpolymere häufig stark temperaturabhängige Eigenschaften aufweisen und dass sich die Temperaturen während der Anwendung deutlich von der Raumtemperatur unterscheiden. Insofern weisen die hier beanspruchten Zahnschienen ein temperaturmäßig deutlich angepassteres Eigenschaftsprofil auf.

Damit verbunden ist insbesondere eine höhere Zähigkeit des Materials bei 35 °C, so dass durch die hier beanspruchten Zahnschienen im Mundraum über längere Zeit deutlich höhere Kräfte aufgebracht werden, als durch die aus dem Stand der Technik bekannten Materialien. Bei Letzteren ist insbesondere festzustellen, dass bei den relevanten Temperaturen in der Mundhöhle deutlich geringere Festigkeiten und/oder geringere Verformbarkeiten resultieren.

Das oben beschrieben und charakterisierte Material zeigt bei der Anwendungstemperatur gleichzeitig einen hohen Modul und eine hohe Zähigkeit, bei reversibler, plastischer Verformbarkeit. Neben der temperaturangepassten Eignung ergibt sich ein weiterer Vorteil durch die Auswahl von Polymeren mit den hier genannten physikalischen Eigenschaften.

Überraschenderweise wurde gefunden, dass Zahnschienen aus einem Polymer mit der oben angegebenen Glasübergangstemperatur, dem oben angegebenen Modul bei 35 °C und dem oben angegebenen Verlustfaktor bei 35 °C bevorzugte Anwendungseigenschaften aufweisen, welche die Akzeptanz und den Tragekomfort im Mundbereich erhöhen.

Das Material ist zäh-elastisch genug, um einfach auf die Zähne gesetzt und wieder entfernt werden zu können. Das Material ist rigide genug, um einen ausreichenden Druck auf die Zähne zur Veränderung der Zahnposition ausüben zu können.

Des Weiteren ergibt sich eine hinreichende Verformung des Materials im Temperaturbereich der Anwendung, so dass auch eine gewisse Anpassung der Zahnschiene an die aktuelle Zahnposition erfolgt. Dies kann in Summe zu einer effizienteren Behandlung von Zahnfehlstellungen führen.

Ein weiterer Vorteil der erfindungsgemäßen Zahnschienen besteht zudem darin, dass diese nicht thermoplastisch sind und deshalb auch bei Temperaturen von > 60 °C wärmeformbeständig sind, das heißt ihre Form bei höheren Temperaturen im unverformten Zustand nicht verändern.

Bevorzugt können höhere Temperaturen sogar dazu benutzt werden leicht verformte Schienen in ihren Ausgangszustand zurück zu formen, da die erfinderischen Polymere einen ausgeprägten Memoryeffekt haben, der die

Druckgeometrie als Formbasis hat. Auch dies ist ein Indiz, dass die mechanischen Eigenschaften der erfindungsgemäßen Zahnschienen im Vergleich zu den Materialien aus dem Stand der Technik temperaturbeständiger sind. Weiterhin vorteilhaft ist, dass die eingesetzten Polymere zur Herstellung der erfindungsgemäßen Zahnschienen transparente Zahnkorrekturschienen liefern können.

Eine kieferorthopädische Zahnschiene im Sinne der Erfindung ist eine Aufsatzform für eine oder mehrere Zähne oder eine gesamte Zahnreihe, wobei die Schiene zur Behandlung von Zahnfehlstellungen geeignet ist. Zur Behandlung der Zahnfehlstellungen wird die Zahnschiene auf die Zähne aufgesetzt und über einen gewissen Behandlungszeitraum getragen. Durch die räumliche Ausgestaltung der Zahnschiene wird auf speziellen Zahnkontaktpunkten Druck ausgeübt, so dass die Zähne von einer Anfangs- hin zu einer geänderten Endposition verschoben werden. Die Größenordnung der Positionsänderung der Zähne ergibt sich dabei als Funktion der Tragedauer und des aufgewendeten Druckes. Insbesondere hat sich ergeben, dass über die erfindungsgemäß eingesetzten Zahnschienen, mit den erfindungsgemäßen mechanischen Eigenschaften, eine höhere Kraft zur Positionierung der Zähne aufgewendet werden kann, so dass innerhalb kürzerer Zeiten bessere Behandlungserfolge erreichbar sind.

Die Zahnschiene umfasst ein vernetztes Polymer oder besteht aus einem solchen. Dies bedeutet, dass die Zahnschiene entweder nur aus den vernetzten Polymer besteht oder dass die erfindungsgemäße Zahnschiene das vernetzte Polymer aufweist. Es ist also möglich, dass die Zahnschiene neben dem Polymer noch weitere Substanzen umfasst. Beispielsweise können neben dem eigentlichen Polymer noch weitere Hilfsstoffe eingesetzt werden, welche dem Fachmann zur Ausbildung vernetzter Polymernetzwerke bekannt sind. Dies können beispielsweise Starter für die Vernetzungsreaktion, Katalysatoren für die Vernetzung, Flexibilisatoren, Farbstoffe, Füllstoffe, Weichmacher oder weitere strukturgebende Substanzen sein, welche ein adaptiertes Eigenschaftsprofil der Zahnschiene ermöglichen. Bevorzugt besteht die Zahnschiene zu > 50 Gew.-%, des Weiteren bevorzugt zu > 70 Gew.-%, weiterhin bevorzugt zu > 80 und ganz besonders bevorzugt > 90 Gew.-% aus dem vernetzten Polymer. Die Gewichtsprozent-Angaben beziehen sich, soweit nicht anders angegeben, immer auf das Gesamtgewicht der Zahnschiene.

Die Glasübergangstemperatur $T_g$ des vernetzten Polymers der Zahnschiene wird in Zugbeanspruchung mittels dynamisch-mechanischer Analyse bei einer Frequenz von 1/s als Peak tan $\delta$ bestimmt und beträgt ≥ 25 °C bis ≤ 60 °C, vorzugsweise ≥ 35 °C bis ≤ 48 °C. Überraschenderweise wurde gefunden, dass solche vernetzte Polymere nach einer Aushärtung besonders geeignet sind, um die in der Anwendung der Zahnkorrekturschiene im Mundraum notwendigen mechanischen Eigenschaften abzubilden.

Der Elastizitätsmodul des vernetzten Polymers der Zahnschiene, bestimmt in Zugbeanspruchung mittels dynamisch-mechanischer Analyse als Speichermodul E' bei einer Frequenz von 1/s bei 35 °C, beträgt ≥ 500 MPa und ≤ 4000 MPa und vorzugsweise ≥ 1000 MPa und ≤ 2500 MPa. Dieser Bereich des Elastizitätsmoduls der Zahnschiene hat sich als besonders geeignet erwiesen um im Rahmen kurzer Behandlungsdauern eine ausreichende Kraft auf die Zähne auszuüben. Dieser Bereich ist also geeignet, um relativ schnell einen verlässlichen Behandlungserfolg zu erreichen.

Der Verlustfaktor tan $\delta$ des vernetzten Polymers der Zahnschiene, gemessen mittels dynamisch-mechanischer Analyse in Zugbeanspruchung bei 35 °C, beträgt ≥ 0,08. Dieser Größenbereich des Verlustfaktors des Materials der Zahnschiene kann zu einem geeigneten zäh-elastischen Verhalten der Zahnschiene insgesamt beitragen. Das zähelastische Verhalten ist dabei eine wichtige Kenngröße, welche sowohl Einfluss auf die Applikationseigenschaften der Zahnschiene wie auch auf die Wechselwirkungen der Zahnschiene mit den Zähnen hat. Bevorzugt kann der Verlustfaktor ≥ 0,1, bevorzugt ≥ 0,12, weiterhin bevorzugt ≤ 0,6 und weiterhin bevorzugt ≤ 0,4 betragen.

Es wurde überraschenderweise gefunden, dass das Auffinden von Rezepturen für vernetzbare Harze, aus welchen die vernetzten Polymere der Zahnschiene mit den gewünschten mechanischen Eigenschaften erhalten werden, auf einfache Weise erfolgen kann. Hierzu werden literaturbekannte Daten der Glasübergangstemperaturen von Homopolymeren der entsprechenden reinen Monomere beziehungsweise polymerisierbaren Bestandteile, insbesondere (Meth)acrylatmonomeren, bereitgestellt.

Eine Rezeptur wird generiert, welche eine Mehrzahl von Monomeren, insbesondere (Meth)acrylatmonomeren, enthält und in welcher die Gewichtsanteile der jeweiligen Monomere festgelegt sind. Die Generierung der Rezeptur kann zweckmäßigerweise auf computerimplementierte Weise erfolgen. Eine theoretische Glasübergangstemperatur für die Rezeptur wird berechnet, indem die Gewichtsanteile der jeweiligen Monomere, beispielsweise ausgedrückt in Gewichts-% (bezogen auf das Gesamtgewicht der polymerisierbaren Bestandteile) mit der Glasübergangstemperatur, welche den Monomeren zugeordnet ist, multipliziert werden und die Ergebnisse summiert werden.

Alternativ zur Summierung der anteilsgewichteten Glasübergangstemperaturen von zwei Monomeren kann die Fox-Gleichung verwendet werden, aus welcher die Glasübergangstemperatur $T_g$ wie folgt berechnet werden kann. Wiedergegeben ist der Fall von zwei verschiedenen Monomeren:

$$1/T_g = w_1/T_{g,1} + w_2/T_{g,2}$$

Mit $w_1$ und $w_2$ als Gewichtsanteile der Komponenten 1 und 2 und $T_{g,1}$ und $T_{g,2}$ als Glasübergangstemperaturen

der aus Komponenten 1 beziehungsweise 2 erhaltenen Homopolymeren.

Selbstverständlich sind weiter verfeinerte Modelle der Vorhersage einer Glasübergangstemperatur eines Copolymers aus mehr als zwei Monomerbestandteilen ebenfalls einsetzbar.

Wenn diese theoretische Glasübergangstemperatur nicht im gewünschten Bereich liegt, wird der Anteil mindestens eines Monomers verändert, bis ein gewünschter Wert erhalten wird. So kann, wenn die theoretische Glasübergangstemperatur niedriger als ein Zielwert ist, der Anteil eines Monomers mit einer höheren Glasübergangstemperatur für das Homopolymer als die derzeitige theoretische Glasübergangstemperatur erhöht werden. In umgekehrter Weise kann eine Absenkung der theoretischen Glasübergangstemperatur erreicht werden.

Wenn die theoretische Glasübergangstemperatur dieser Ausgangsrezeptur den gewünschten Bereich erreicht hat, werden ausgehend von dieser Rezeptur wie zuvor beschrieben eine vorbestimmte Anzahl von Rezepturen generiert, welche eine theoretische Glasübergangstemperatur aufweisen, die um einen vorbestimmten Betrag höher oder niedriger als diejenige der Ausgangsrezeptur liegen. Beispielsweise können 10 Rezepturen generiert werden, welche eine theoretische Glasübergangstemperatur im Bereich zwischen 10 °C unterhalb und 30 °C oberhalb der theoretischen Glasübergangstemperatur der Ausgangsrezeptur aufweisen. Zweckmäßigerweise werden bei der Generierung der Rezepturen möglichst wenige Parameter verändert, beispielsweise die relativen Anteile von zwei Monomeren mit unterschiedlicher Glasübergangstemperatur ihrer Homopolymere.

Diese so generierten Rezepturen können dann im Labor hergestellt und die aus ihnen erhaltenen vernetzten Polymere in Bezug auf ihre mechanischen Eigenschaften geprüft werden. Eine finale Rezeptur kann durch Interpolation oder Regressionsanalyse mit der Glasübergangstemperatur als zu erreichende Größe erhalten werden.

Es hat sich weiterhin überraschend gezeigt, dass wenn die Glasübergangstemperatur eines aus einer derart erhaltenen finalen Rezeptur hergestellten Polymers innerhalb der erfindungsgemäßen Spezifikationen liegen, der Elastizitätsmodul bei 35 °C und der Verlustfaktor tan $\delta$ bei 35 °C wahrscheinlich ebenfalls die erfindungsgemäßen Spezifikationen erfüllen. Dieses reduziert in der Praxis den Erprobungsaufwand für eine geeignete Rezeptur erheblich.

In einer Ausgestaltung der Zahnschiene kann das vernetzte Polymer ein vernetztes Polyisocyanat oder Polyurethan umfassen.

Gemäß einer weiteren Ausführungsform ist das vernetzte Polymer erhältlich aus der Vernetzung eines Harzes umfassend die folgenden Komponenten, wobei die Mengen in Gewichts-%, bezogen auf das Gesamtgewicht des Harzes angegeben sind und sich zu ≥ 86 Gewichts-% bis ≤ 100 Gewichts-% addieren:

| | |
|---|---|
| Isocyanuratgruppen enthaltendes Urethan(meth)acrylat | 15 - 20 |
| Alkandioldi(meth)acrylat | 1-5 |
| Monofunktionelles (Meth)Acrylat mit einer Glasübergangstemperatur des hieraus erhaltenen Homopolymers von ≤ 0 °C | 20 - 30 |
| Monofunktionelles (Meth)Acrylat mit einer Glasübergangstemperatur des hieraus erhaltenen Homopolymers von ≥ 60 °C | 50 - 60 |

oder das vernetzbare Harze umfasst die folgenden Komponenten, wobei die Mengen in Gewichts-%, bezogen auf das Gesamtgewicht des Harzes angegeben sind und sich zu ≥ 90 Gewichts-% bis ≤ 100 Gewichts-% addieren:

| | |
|---|---|
| Uretdiongruppen enthaltendes Urethan(meth)acrylat | 10 - 15 |
| Monofunktionelles Methacrylat eines Terpenalkohols | 5 - 10 |
| Monofunktionelles (Meth)Acrylat mit einer Glasübergangstemperatur des hieraus erhaltenen Homopolymers von ≤ 0 °C | 20 - 35 |
| Monofunktionelles (Meth)Acrylat mit einer Glasübergangstemperatur des hieraus erhaltenen Homopolymers von ≥ 60 °C | 55-60 |

Die Harze können weiterhin Additive wie Photoinitiatoren, Radikalstarter, Inhibitoren, Stabilisatoren und dergleichen enthalten.

Geeignete Isocyanuratgruppen enthaltende Urethan(meth)acrylate sind zum Beispiel erhältlich, indem aliphatische Diisocyanate, insbesondere Pentamethylendiisocyanat, Hexamethylendiisocyanat und Isophorondiisocyanat, oder eine Mischung aus mindestens zwei hiervon, zu Isocyanuraten trimerisiert werden und anschließend mit Hydroxyalkyl(meth)acrylaten wie Hydroxyethylmethacrylat (HEMA) oder Hydroxypropylacrylat zum Urethan umgesetzt werden.

Geeignete Uretdiongruppen enthaltende Urethan(meth)acrylate sind zum Beispiel erhältlich, indem aliphatische

Diisocyanate, insbesondere Pentamethylendiisocyanat, Hexamethylendiisocyanat und Isophorondiisocyanat, oder eine Mischung aus mindestens zwei hiervon, zu Uretdionen dimerisiert werden und mit Polyolen (insbesondere Polyetherpolyolen) zu NCO-terminierten Prepolymeren umgesetzt werden. Diese Prepolymere können dann mit Hydroxyalkyl(meth)acrylaten wie Hydroxyethylmethacrylat (HEMA) oder Hydroxypropylacrylat zum Urethan umgesetzt werden.

In einer weiteren Ausführungsform der Zahnschiene kann das vernetzte Polymer einen Isocyanuratanteil, ermittelt über $^{13}$C-NMR, von ≥ 3% aufweisen. Insbesondere der Einsatz vernetzter Polymere mit einem hinreichenden Isocyanuratanteil in oben angegebenen Bereich kann zu den bevorzugten temperaturabhängigen Eigenschaften der Zahnschiene beitragen. Insbesondere kann dieser Isocyanuratanteil zu der bevorzugten Festigkeit, ausgedrückt durch das erfindungsgemäße Speichermodul, führen. Weiter bevorzugte Isocyanuratanteile können bevorzugt bei > 5 %, des Weiteren bevorzugt bei > 8 %, weiterhin bevorzugt > 10 % betragen. Die %-Angaben beziehen sich dabei auf den Gewichts-Anteil des Isocyanuratrings.

Weiterhin kann in einem bevorzugten Aspekt der Zahnschiene das vernetzte Polymer einen Urethananteil ermittelt über $^{13}$C NMR von ≥ 3% aufweisen. Insbesondere der Einsatz vernetzter Polymere mit einem hinreichenden Urethananteil in oben angegebenen Bereich kann zu den bevorzugten temperaturabhängigen Eigenschaften der Zahnschiene beitragen. Insbesondere kann dieser Urethananteil zu der bevorzugten Festigkeit und Zähelastizität, ausgedrückt durch das erfindungsgemäße Speichermodul und den erfindungsgemäßen tan $\delta$, führen. Weiter bevorzugte Urethananteile können bevorzugt bei > 5 %, des Weiteren bevorzugt bei > 7 %, weiterhin bevorzugt > 8 % betragen. Die %-Angaben beziehen sich dabei auf den Gewichts-Anteil der Urethangruppe.

In einer bevorzugten Ausführungsform der Zahnschiene weist das vernetzte Polymer einen Brechungsindex, gemessen mit einem Abbe Refraktometer, von >1,48 RI und < 1,58 RI auf. Dieser Bereich des Brechungsindex, in Kombination mit einer niedrigen Wasserquellung, bevorzugt von < 3 Gew.-%, oder bevorzugt von < 2 Gew.-%, oder bevorzugt von < 1 Gew.-% und oder bevorzugt von < 0,5 Gew.-% und einer guten Chemikalienbeständigkeit bzw. Fleckbeständigkeit gegen typische Nahrungs- und Genussmittel wie Kaffee, Senf, Rotwein mit einer Bewertung von bevorzugt ≥ 3 bevorzugt ≥ 4 und besonders bevorzugt = 5 (Bewertung nach Auftrag der Substanz auf die Produktoberfläche, nach Einwirkzeit von 10 min, anhand der Verfärbung des Produktes: Bewertung mit 5 = ungefärbt und 0 = stark gefärbt in Analogie zu Lackbeständigkeitsprüfungen), kann dazu beitragen, dass die erfindungsgemäße Zahnschiene unter den physikalischen und chemischen Bedingungen der Mundhöhle, d.h. im Temperaturbereich der Mundhöhle und unter den Feuchtebedingungen der Mundhöhle, nahezu nicht sichtbar ist. Ohne durch die Theorie gebunden zu sein ergibt sich das bevorzugt aus der Wahl des vernetzten Polymeren, welches zudem die erfindungsgemäßen mechanischen Eigenschaften aufweist. Diese Bereiche sind dazu geeignet, das Tragen der Schiene weniger auffällig zu gestalten und insofern kann die Motivation des Anwenders zum Tragen der Zahnschiene gesteigert werden. Dies kann dazu beitragen, dass der gewünschte Behandlungserfolg schneller erreicht wird. In weiteren Ausführungsformen kann der Brechungsindex > 1,49 und < 1,56, weiterhin bevorzugt > 1,495 und < 1,54 und des Weiteren bevorzugt < 1,5 und < 1,53 betragen. Die Messung des Brechungsindex erfolgt bei einer Temperatur von 35 °C.

Innerhalb einer weiteren Charakteristik der Zahnschiene kann das vernetzte Polymer eine mittlere Netzbogenlänge nach Flory und Huggins von > 300 g/mol und < 5000 g/mol, aufweisen. Diese Bereiche der Netzbogenlängen der vernetzten Polymere haben sich zum Erhalt ausreichend zähelastischer Eigenschaften der Zahnschiene als besonders geeignet erwiesen. Innerhalb dieses Bereiches kann die Zahnschiene die erforderliche Rigidität und zudem die in der Anwendung nötige Zäh-Elastizität aufweisen. Dies kann zu bevorzugten Anwendungseigenschaften, wie beispielsweise leichtes Einsetzen der Zahnschiene und eine schnelle Drehpositionierung der Zähne beitragen. Die mittlere Netzbogenlänge kann dabei durch eine Quellungsmessung in Aceton bestimmt werden. Beim Quellen eines Netzwerkes mit einem Lösungsmittel führt dessen Eindringen zu einer Volumenzunahme (attraktive Wechselwirkung zwischen Lösemittel und Polymermatrix), und die Gibbsche Mischungsenthalpie ΔGm steigt. Eine entropiegetriebene Rückstellkraft ΔGel wirkt diesem Prozess entgegen. Beim Erreichen eines Gleichgewichtszustandes wird die Gibbsche freie Energie ΔG Null. ΔGm lässt sich mit Hilfe der Flory-Huggins-Gleichung bestimmen, ΔGm folgt aus der Gausschen Netzwerktheorie. Aus der Bedingung ΔG=0 für den Gleichgewichtszustand der Quellung folgt die Flory-Rehner-Gleichung:

$$M_C = \frac{\rho V_{m1}\left(\frac{\emptyset_2}{2} - \emptyset_2^{\frac{1}{3}}\right)}{\ln(1-\emptyset_2) + \emptyset_2 + (\chi \emptyset_2^{\,2})}$$

Mit der Dichte des Polymers und dem Hugginsschen Wechselwirkungsparameter, lässt sich anhand von Quellungsexperimenten zur Bestimmung des Volumenanteils des Polymers das Molekulargewicht der mittleren Netzbogenlänge MC bestimmen.

Alternativ kann die mittlere Netzbogenlänge Mc sowie die Vernetzungsdichte v aus dem Minimum im Gummiplateau einer DMA Messung bei 1 Hz und 0,1% Auslenkung oberhalb vom Tg des Produktes bestimmt werden.

$$\mathrm{Mc} = \frac{\rho}{\nu} = \frac{3\rho RT}{E'_{min}}$$

$$\nu = \frac{E'_{min}}{3RT}$$

$$Mc = Netzbogenlänge\ [\frac{g}{mol}]$$

*$\rho$ = Dichte des Polymers* $[\frac{kg}{m^3}]$

*R = molare Gaskonstante* $[\frac{J}{mol*K}]$

*T = absolute Temperatur [K]*

*E' = Speichermodul* [-]

*$\nu$* = molare Vernetzungsdichte $[\frac{\mathrm{mol}}{\mathrm{cm}^3}]$

Bevorzugt kann die Netzbogenlänge > 400 g/mol und < 2000 g/mol, weiterhin bevorzugt >500 g/mol und < 1600 g/mol, des Weiteren bevorzugt > 550 g/mol und < 1400 g/mol und > 600 g/mol und <1200 g/mol betragen.

Im Rahmen einer weiteren Ausführungsform der Zahnschiene kann das Polymer ein transparentes Polymer mit einer Lichttransmission gemessen in einem UV-VIS Spektrometer an einer Probe mit einer Dicke von 1 mm im Wellenlängenbereich von 400-800 nm von >50% sein. Diese Transmission kann dabei besonders bevorzugt mit einem b* Wert im L*a*b* im CIELab-Farbenraum von < 50, bevorzugt < 30, bevorzugt <20 kombiniert sein. Gerade die Auswahl transparenter Materialien, welche nach der Fertigung transparent und farblos sind, kann die Trageneigung des Anwenders deutlich steigern und so innerhalb kürzerer Zeit zu einem besseren Behandlungserfolg beitragen. Bevorzugt kann die Transmission in oben angegebenen Wellenlängenbereich > 60%, bevorzugt > 70%, weiterhin bevorzugt > 80% und ebenfalls bevorzugt > 90% betragen. Die CIELab-Werte können über handelsübliche Geräte bestimmt werden.

Innerhalb einer weiteren Ausführungsform der Zahnschiene kann das Polymer ein transparentes Polymer enthaltend Polyurethane und/oder Polysilicone sein und eine Abbe Zahl von > 20 aufweisen. Neben der reinen Transmission hat sich überraschenderweise für die vernetzten Polymere auch die Abbe-Zahl als wesentlicher Parameter für die nutzerbezogene "Sichtbarkeit" der Zahnschiene unter Tragebedingungen erweisen. Zahnschienen mit den erfindungsgemäßen Abbe-Zahlen sind dabei besonders unauffällig und können so zu einer erhöhten Anwendungszeit durch den Nutzer beitragen. Bevorzugt kann die Abbe-Zahl für oben angegebene Zahnschiene > 25, bevorzugt >30, weiterhin bevorzugt > 35 und ebenfalls bevorzugt > 38 betragen.

Ein weiterer Gegenstand der Erfindung ist ein Verfahren zur Herstellung einer kieferorthopädischen Zahnschiene, umfassend die Schritte: i) Auswahl eines vernetzbare Harzes und ii) Formen der Zahnschiene durch Vernetzen des in Schritt i) ausgewählten vernetzbaren Harzes unter Ausbildung eines vernetzten Polymers, wobei die Auswahl in Schritt i) das Kriterium umfasst, dass ein nach Vernetzung des vernetzbaren Harzes erhaltenes vernetztes Polymer eine Glasübergangstemperatur $T_g$, bestimmt mittels dynamisch-mechanischer Analyse bei einer Frequenz von 1/s DM A als Peak tan $\delta$, von ≥ 25 °C und ≤ 60 °C (vorzugsweise ≥ 35 °C bis ≤ 48 °C), ein Elastizitätsmodul, bestimmt mittels dynamisch-mechanischer Analyse als Speichermodul E' bei einer Frequenz von 1/s bei 35 °C, von ≥ 500 MPa und ≤ 4000 MPa (vorzugsweise ≥ 1000 MPa und ≤ 2500 MPa) und einen Verlustfaktor tan $\delta$, bestimmt mittels dynamisch-mechanischer Analyse bei einer Frequenz von 1/s bei 35 °C, von ≥ 0,08 (vorzugsweise ≥ 0,1, mehr bevorzugt ≥ 0,12, weiterhin bevorzugt ≤ 0,6 und weiterhin bevorzugt ≤ 0,4 aufweist.

Das vernetzbare Harz enthält ein erstes Monomer und ein zweites Monomer, wobei das erste Monomer ein (meth)acrylisches Monomer ist, dessen Homopolymer eine Glasübergangstemperatur, bestimmt mittels dynamisch-mechanischer Analyse bei einer Frequenz von 1/s als Peak tan $\delta$, von ≤ 0 °C aufweist und das zweite Monomer ein (meth)acrylisches Monomer oder ein styrolisches Monomer ist, dessen Homopolymer eine Glasübergangstemperatur, bestimmt mittels dynamisch-mechanischer Analyse bei einer Frequenz von 1/s als Peak tan $\delta$, von ≥ 60 °C aufweist und wobei das erste Monomers in einem Anteil von ≥ 5 bis ≤ 40 Gewichts-%, bezogen auf das Gesamtgewicht des Harzes, vorliegt und das zweite Monomer in einem Anteil von ≥ 20 bis ≤ 80 Gewichts-%, bezogen auf das Gesamtgewicht des Harzes, vorliegen. Die genannten Gewichts-%-Angaben addieren sich zu ≤ 100 Gewichts-%. Das erste Monomer

kann als sogenanntes "low $T_g$ monomer" und das zweite als "high $T_g$ monomer" bezeichnet werden. Solche Monomere und die entsprechenden Glasübergangstemperaturen sind bekannt und kommerziell zugänglich. Beispiele für das erste Monomer sind n-Butylacrylat und sec-Butylacrylat. Beispiele für das zweite Monomer sind Isobornylacrylat (IBOA) und Isobornylmethacrylat (IBOMA).

Vorzugsweise umfasst das in Schritt i) ausgewählte vernetzbare Harz die folgenden Komponenten, wobei die Mengen in Gewichts-%, bezogen auf das Gesamtgewicht des Harzes angegeben sind und sich zu ≥ 86 Gewichts-% bis ≤ 100 Gewichts-% addieren:

| | |
|---|---|
| Isocyanuratgruppen enthaltendes Urethan(meth)acrylat | 15 - 20 |
| Alkandioldi(meth)acrylat | 1-5 |

| | |
|---|---|
| Monofunktionelles (Meth)Acrylat mit einer Glasübergangstemperatur des hieraus erhaltenen Homopolymers von ≤ 0 °C | 20 - 30 |
| Monofunktionelles (Meth)Acrylat mit einer Glasübergangstemperatur des hieraus erhaltenen Homopolymers von ≥ 60 °C | 50 - 60 |

oder das in Schritt i) ausgewählte vernetzbare Harze umfasst die folgenden Komponenten, wobei die Mengen in Gewichts-%, bezogen auf das Gesamtgewicht des Harzes angegeben sind und sich zu ≥ 90 Gewichts-% bis ≤ 100 Gewichts-% addieren:

| | |
|---|---|
| Uretdiongruppen enthaltendes Urethan(meth)acrylat | 10 - 15 |
| Monofunktionelles Methacrylat eines Terpenalkohols | 5 - 10 |
| Monofunktionelles (Meth)Acrylat mit einer Glasübergangstemperatur des hieraus erhaltenen Homopolymers von ≤ 0 °C | 20 - 35 |
| Monofunktionelles (Meth)Acrylat mit einer Glasübergangstemperatur des hieraus erhaltenen Homopolymers von ≥ 60 °C | 55-60 |

Details zu den vernetzbaren Harzen und bevorzugte Zusammensetzungen wurden bereits im Zusammenhang mit der Zahnschiene weiter oben im Text aufgeführt. Sie gelten genauso für die vernetzbaren Harze im erfindungsgemäßen Verfahren und werden zur Vermeidung von Wiederholungen nicht erneut wiedergegeben.

Gemäß einer Ausführungform wird in Schritt ii) die Zahnschiene durch Vernetzen des vernetzbaren Harzes in einer zu der Zahnschiene korrespondierenden Gießform geformt.

Gemäß einer weiteren Ausführungsform wird in Schritt ii) die Zahnschiene mittels eines additiven Herstellungsverfahrens geformt. Beispiele für geeignete additive Herstellungsverfahren ("3D-Druck") sind DLP (Dynamic Light Processing), CLIP (Continuous Liquid Interface Production) Inkjetverfahren oder SLA (laserbasierte Stereolithografie).

Das erfindungsgemäße Verfahren kann beispielsweise ein Verfahren zur Herstellung einer kieferorthopädischen Zahnschiene mittels eines 3D-Druckverfahrens sein, wobei ein Polymer enthaltend actinisch polymerisierbare Doppelbindungen in Form einer kieferorthopädischen Zahnschiene ausgedruckt und polymerisiert wird.

Der Stand der Technik liefert keine Hinweise zur Materialentwicklung von geeigneten flüssigen actinisch härtbaren Materialien (Harzformulierungen) für den Einsatz in 3D Druckverfahren zur Herstellung von Zahnkorrekturschienen auf Basis hochvernetzter Polymere. Veröffentlichte Daten diskutieren Materialeigenschaften immer nur in Bezug auf die Eigenschaften bei Raumtemperatur und lassen die Gebrauchseigenschaften bei erhöhter Temperatur unberücksichtigt.

Bevorzugt können die erfindungsgemäß einsetzbaren vernetzbare Harze bei einer typischen Viskosität von < 10.000 mPas, bevorzugt < 5000 mPas, besonders bevorzugt < 1000 mPas bei Verarbeitungstemperatur von >5 °C und < 150 °C, bevorzugt > 15 und < 120 °C, besonders bevorzugt > 20 und < 110 °C und ganz besonders bevorzugt > 30 und < 100 °C im 3D-Drucker verarbeitet werden.

Ein erfindungsgemäßes Verfahren zur Herstellung eines Gegenstandes aus einem Vorläufer kann die folgenden Schritte umfassen:

I) Abscheiden eines radikalisch vernetzten Polymers auf einem Träger, so dass eine Lage eines mit dem Träger verbundenen Aufbaumaterials erhalten wird, welche einem ersten ausgewählten Querschnitt des Vorläufers entspricht;

II) Abscheiden eines radikalisch vernetzten Polymers auf eine zuvor aufgetragene Lage des Aufbaumaterials, so dass eine weitere Lage des Aufbaumaterials erhalten wird, welche einem weiteren ausgewählten Querschnitt des Vorläufers entspricht und welche mit der zuvor aufgetragenen Lage verbunden ist;

III) Wiederholen des Schritts II), bis der Vorläufer gebildet ist;

wobei das Abscheiden eines radikalisch vernetzten Polymers wenigstens in Schritt II) durch Einwirken von Energie auf einen ausgewählten Bereich eines radikalisch vernetzbaren Harzes, entsprechend dem jeweils ausgewählten Querschnitt des Gegenstandes, erfolgt und wobei das radikalisch vernetzbare Polymer eine Viskosität (bei Verarbeitungstemperatur, DIN EN ISO 2884-1) von $\geq$ 5 mPas bis $\leq$ 100000 mPas aufweist.

Das radikalisch vernetzbare Harz kann in einer Variante eine härtbare Komponente enthalten, in der mit einem Blockierungsmittel blockierte NCO-Gruppen, Verbindungen mit mindestens zwei Zerewitinoff-aktiven H-Atomen sowie olefinische C=C-Doppelbindungen vorliegen, wobei das Blockierungsmittel ein Isocyanat ist oder das Blockierungsmittel derart ausgewählt ist, dass nach Deblockierung der NCO-Gruppe keine Freisetzung des Blockierungsmittels als freies Molekül stattfindet.

Es kann nach Schritt III) weiterhin Schritt IV) durchgeführt werden:

IV) Behandeln des nach Schritt III) erhaltenen Vorläufers bei Bedingungen, die ausreichen, um im radikalisch vernetzten Polymer des erhaltenen Vorläufers vorliegende NCO-Gruppen zumindest teilweise zu deblockieren und die dadurch erhaltenen funktionellen Gruppen mit Verbindungen mit mindestens zwei Zerewitinoff-aktiven H-Atomen zur Reaktion zu bringen, so dass der Gegenstand erhalten wird.

In diesem Verfahren kann der Gegenstand in zwei Herstellungsabschnitten erhalten werden. Der erste Herstellungsabschnitt kann als Aufbauabschnitt angesehen werden. Dieser Aufbauabschnitt lässt sich mittels strahlenoptischer additiver Fertigungsverfahren wie der Stereolithographie oder dem DLP (digital light processing)-Verfahren oder auch mittels Inkjet-Druckverfahren kombiniert mit Strahlenvernetzung realisieren und ist Gegenstand der Schritte I), II) und III). Der zweite Herstellungsabschnitt kann als Härtungsabschnitt angesehen werden und ist Gegenstand des Schritts IV). Hier wird der nach dem Aufbauabschnitt erhaltene Vorläufer oder intermediäre Gegenstand ohne seine Form weiter zu verändern in einen mechanisch dauerhafteren Gegenstand überführt. Das Material, aus dem der Vorläufer im additiven Fertigungsverfahren erhalten wird, kann auch als "Aufbaumaterial" bezeichnet werden.

Das Blockierungsmittel kann ausgewählt sein aus der Gruppe bestehend aus organischen Isocyanaten, Lactamen, Glycerincarbonat, einer Verbindung gemäß der allgemeinen Formel (I):

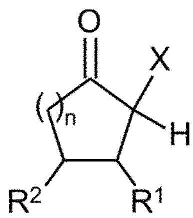
(I)

in welcher X eine elektronenziehende Gruppe, $R^1$ und $R^2$ unabhängig voneinander die Reste H, Ci-$C_{20}$-(Cyclo)alkyl, $C_6$-$C_{24}$-Aryl, $C_1$-$C_{20}$-(Cyclo)alkylester oder -amid, $C_6$-$C_{24}$-Arylester oder -amid, gemischt aliphatisch/aromatische Reste mit 1 bis 24 Kohlenstoffatomen, die auch Teil eines 4 bis 8-gliedrigen Ringes sein können, darstellen und n eine ganze Zahl von 0 bis 5 ist, oder einer Kombination aus mindestens zwei hiervon.

Bevorzugte Verbindungen der allgemeinen Formel (I) sind Cyclopentanon-2-carboxymethylester und -carboxyethylester, Cyclopentanon-2-carbonsäurenitril, Cyclohexanon-2-carboxymethylester und -carboxyethylester oder Cyclopentanon-2-carbonylmethyl. Besonders bevorzugt sind Cyclopentanon-2-carboxymethylester und -carboxyethylester sowie Cyclohexanon-2-carboxymethylester und-carboxyethylester. Die Cyclopentanonsysteme sind technisch leicht durch eine Dieckmann-Kondensation von Adipinsäuredimethylester oder Adipinsäurediethylester erhältlich. Cyclohexanon-2-carboxymethylester kann durch Hydrierung von Salicylsäuremethylester hergestellt werden.

In einer weiteren Variante dieses Verfahrens sind in der härtbaren Komponente die Verbindungen mit mindestens zwei Zerewitinoff-aktiven H-Atomen ausgewählt aus der Gruppe bestehend aus Polyaminen, Polyolen oder einer Kombination hiervon. Dies können beispielsweise niedermolekulare Diole (z. B. 1,2-Ethandiol, 1,3- bzw. 1,2-Propandiol, 1,4-Butandiol), Triole (z.B. Glycerin, Trimethylolpropan) und Tetraole (z. B. Pentaerythrit) sein, kurzkettige Polyamine aber auch höhermolekulare Polyhydroxyverbindungen wie Polyetherpolyole, Polyesterpolyole, Polycarbonatpolyole, Polysiloxanpolyole, Polyamine und Polyetherpolyamine sowie Polybutadienpolyole.

Die härtbare Verbindung ist vorzugsweise eine Verbindung, die erhältlich ist aus der Dimerisierung eines Diisocyanats zu einem NCO-terminierten Uretdion, gefolgt von der Umsetzung der NCO-Gruppen mit einem Hydroxyalkyl(meth)acrylat.

In einer weiteren Variante dieses Verfahrens umfasst in Schritt IV) das Behandeln des nach Schritt III) erhaltenen Vorläufers bei Bedingungen, die ausreichen, um im radikalisch vernetzten Polymer des erhaltenen Vorläufers vorliegende NCO-Gruppen zumindest teilweise zu deblockieren und die dadurch erhaltenen funktionellen Gruppen mit Verbindungen mit mindestens zwei Zerewitinoff-aktiven H-Atomen zur Reaktion zu bringen, ein Erwärmen des Körpers auf eine Temperatur von ≥ 60 °C. Vorzugsweise beträgt diese Temperatur ≥ 80 °C bis ≤ 250 °C, mehr bevorzugt ≥ 90 °C bis ≤ 190 °C. Die gewählte Temperatur oder der gewählte Temperaturbereich in Schritt IV) kann beispielsweise für ≥ 5 Minuten bis ≤ 48 Stunden, bevorzugt ≥ 15 Minuten bis ≤ 24 Stunden und mehr bevorzugt ≥ 1 Stunde bis ≤ 12 Stunden gehalten werden.

Alternativ kann nach Schritt III) weiterhin der folgende Schritt IV) durchgeführt werden:
IV) Behandeln des nach Schritt III) erhaltenen Vorläufers bei Bedingungen, die ausreichen, um im radikalisch vernetzten Polymer des erhaltenen Vorläufers vorliegende NCO-Gruppen zumindest teilweise zu Isocyanurat-Gruppen zu trimerisieren, so dass der Gegenstand erhalten wird.

Das Behandeln in Schritt IV) kann im einfachsten Fall ein Lagern bei Raumtemperatur (20 °C) sein. Es ist auch möglich, bei einer Temperatur oberhalb der Raumtemperatur zu lagern. Während des Schritts IV) reagieren die NCO-Gruppen miteinander unter weiterer Vernetzung des zuvor schon radikalisch vernetzten Materials. Diese Reaktion führt zumindest teilweise zur Trimerisierung zu Isocyanurat-Gruppen. Es ist erfindungsgemäß eingeschlossen, dass auch Uretdion-, Allophanat-, Urea-, Urethan-, Biuret-, Iminooxadiazindion- und/oder Oxadiazintriongruppen aus den NCO-Gruppen gebildet werden können. Solche Nebenreaktionen können gezielt eingesetzt werden, um beispielsweise die Glasübergangstemperatur $T_g$ des erhaltenen Materials zu beeinflussen.

Ein weiteres Verfahren zur Herstellung der Zahnschiene aus einem Aufbaumaterial, wobei das Aufbaumaterial radikalisch vernetzbare Gruppen, NCO-Gruppen sowie Gruppen mit Zerewitinoff-aktiven H-Atomen umfasst und der Gegenstand ein dreidimensionaler Gegenstand und/oder eine Schicht ist, zeichnet sich dadurch aus, dass während und/oder nach der Herstellung des Gegenstands das Aufbaumaterial auf eine Temperatur von ≥ 50 °C erwärmt wird und dass das Aufbaumaterial eine oder mehrere der folgenden zyklischen Zinnverbindungen umfasst:
4,12-Di-n-butyl-1,7,9,15-tetraoxa-4,12-diaza-8-stannaspiro[7.7]pentadecan, 4,12-Di-n-butyl-2,6,10,14-tetramethyl-1,7,9,15-tetraoxa-4,12-diaza-8-stannaspiro[7.7]pentadecan, 2,4,6,10,12,14-Hexamethyl-1,7,9,15-tetraoxa-4,12-diaza-8-stannaspiro[7.7]pentadecan, 4,12-Di-n-octyl-2,6,10,14-tetramethyl-1,7,9,15-tetraoxa-4,12-diaza-8-stannaspiro[7.7]pentadecan, 4,12-Di-n-octyl-1,7,9,15-tetraoxa-4,12-diaza-8-stannaspiro[7.7]pentadecan, 4,12-Dimethyl-1,7,9,15-tetraoxa-4,12-diaza-8-stannaspiro[7.7]pentadecan, 1,1-Dichloro-5-methyl-5-aza-2,8-dioxa-1-stannacyclooctan, 1,1-Diisopropyl-5-methyl-5-aza-2,8-dioxa-1-stannacyclooctan, 1,1-Dibenzoyl-3,3,7,7-tetramethyl 5-n-octyl-5-aza-2,8-dioxa-1-stannacyclooctan, 1,1-Dibenzoyl- 5-n-octyl-5-aza-2,8-dioxa-1-stannacyclooctan, 1,1 -Bis(p-dodecylphenylsulfonyl)- 5-n-octyl-5-aza-2,8-dioxa-1-stannacyclooctan, 2-Benzoyloxy-6-octyl-4,8-dioxo-1,3,6,2-dioxazastannocan-2-ylbenzoat oder Mischungen davon.

Diese Zinnverbindungen sind thermisch labil. Unterhalb einer bestimmten Temperatur weisen sie keine technisch sinnvolle katalytische Aktivität für die Reaktion von NCO-Gruppen mit funktionellen Gruppen, welche Zerewitinoff-aktive H-Atome tragen, auf. Insbesondere seien hierbei Urethanisierungen und Harnstoffbildungen zu nennen. Oberhalb einer bestimmten Temperatur steigt jedoch die katalytische Aktivität stark an. Ohne auf eine Theorie beschränkt zu sein wird angenommen, dass dann die Liganden vom Sn-Zentrum ganz oder teilweise dissoziieren und daher das Sn-Zentrum als Katalysator zur Verfügung steht. Insofern lässt sich von thermisch latenten Katalysatoren sprechen. Dadurch, dass die im Aufbaumaterial vorliegenden NCO-Gruppen unterhalb dieser Temperatur nicht abreagieren, kann das Aufbaumaterial auch leicht wiederverwertet werden. Erfindungsgemäß wird zur Aktivierung des Sn-Katalysators auf eine Temperatur von ≥ 50 °C, vorzugsweise ≥ 65 °C, mehr bevorzugt ≥ 80 °C, besonders bevorzugt ≥ 80 °C bis ≤ 200 °C erwärmt, so dass nach erfolgter Reaktion der NCO-Gruppen der Gegenstand erhalten wird. Das Erwärmen kann für eine Zeitspanne von ≥ 1 Minute, bevorzugt ≥ 5 Minuten, mehr bevorzugt ≥ 10 Minuten bis ≤ 24 Stunden bevorzugt ≤ 8 Stunden, besonders bevorzugt < 4 Stunden, erfolgen.

In einer bevorzugten Ausgestaltung des Verfahrens weist das Polymer freie Isocyanatgruppen gemessen mittels $^{13}C$ NMR in einer Konzentration ≥ 1 Gew.-% bezogen auf das Polymer auf. Insbesondere die Konzentration an freien Isocyanatgruppen kann die mechanischen Eigenschaften des ausreagierten Materials der Zahnschiene günstig beeinflussen und zu einer verbesserten Elastizität der Zahnschiene unter Anwendungsbedingungen beitragen. In einer bevorzugten Ausführungsform kann das Polymer freie Isocyanatgruppen in einer Konzentration ≥ 1,5, bevorzugt ≥ 2,0, des Weiteren bevorzugt ≥ 2, 5 und weiterhin bevorzugt ≥ 3,0 Gew.-% enthalten.

Innerhalb einer weiteren bevorzugten Ausführungsform des Verfahrens weist das Polymer Uretdiongruppen gemessen mittels $^{13}C$ NMR in einer Konzentration ≥ 1 Gew.-% bezogen auf das Polymer auf. Insbesondere die Konzentration an Uretdiongruppen kann die mechanischen Eigenschaften des ausreagierten Materials der Zahnschiene günstig beeinflussen und zu einer verbesserten Elastizität der Zahnschiene unter Anwendungsbedingungen beitragen. In einer bevorzugten Ausführungsform kann das Polymer Uretdiongruppen in einer Konzentration ≥ 1,5, bevorzugt ≥ 2,0, des Weiteren bevorzugt ≥ 2,5 und weiterhin bevorzugt ≥ 3,0 Gew.-% enthalten.

In einer bevorzugten Ausgestaltung des Verfahrens weist das Polymer freie Alkoholgruppen gemessen mittels $^{13}C$ NMR in einer Konzentration ≥ 0,5 Gew.-% bezogen auf die Gesamtmasse des Polymer auf. Insbesondere die Konzentration freier Alkoholgruppen im Polymer kann die mechanischen Eigenschaften des ausreagierten Materials der Zahnschiene günstig beeinflussen und zu einer verbesserten Elastizität der Zahnschiene unter Anwendungsbedingungen beitragen. In einer bevorzugten Ausführungsform enthält das Polymer freie Alkoholgruppen in einer Konzentration ≥ 1,0 Gew.-%, bevorzugt ≥ 1,5 Gew.-%, oder bevorzugt > 2,0 Gew.-% oder bevorzugt ≥ 2,5 Gew.-%, bezogen auf die Gesamtmasse des Harz.

Im Rahmen eines weiteren Aspektes des Verfahrens kann das Polymer während und/oder nach dem Druckprozess durch mindestens eine weitere, nicht radikalische Vernetzung vernetzt werden, sich die Netzbogenlänge dabei um mindestens 20% verringern, der $T_g$ um mindestens 3 °C, der Modul um mindestens 15% und die Bruchfestigkeit um mindestens 10% steigen, bezogen auf die Eigenschaften des Polymers vor dem Druckprozess. Es hat sich zur Herstellung der erfindungsgemäßen Zahnschienen als besonders günstig erwiesen, dass die mechanische Festigkeit der Zahnschiene über einen zweistufigen Prozess erhalten wird. Dies kann die Freiheitsgrade im 3D-Druckprozess von den mechanischen Eigenschaften der ausreagierten Zahnschiene entkoppeln, sodass die erfindungsgemäßen mechanischen Eigenschaften der Zahnschiene bei Anwendungstemperatur leichter erhältlich sind. Bevorzugt kann die Härtung des erfindungsgemäßen Polymers zum Produkt innerhalb eines 2-stufigen Prozesses erfolgen, wobei zuerst ein Grünkörper im 3D Drucker erzeugt wird, welcher im Anschluss entnommen, von nicht umgesetzten Polymer gereinigt und in einem zweiten Schritt mittels Strahlen und/oder thermisch nachgehärtet wird. In einer bevorzugten Ausführungsform wird dabei der zu härtende Grünkörper für einen Zeitraum von ≥ 1 min bis ≤ 72h, bevorzugt ≥ 5 min bis ≤ 24h, oder bevorzugt ≥ 10 min bis ≤ 12h, auf eine Temperatur von ≥ 50 °C bis ≤ 250 °C, bevorzugt ≥ 80 °C ≤ 200 °C, oder bevorzugt ≥ 100 °C ≤ 180 °C aufgeheizt.

Die Netzbogenlänge wird bevorzugt über den 2-stufigen Prozess um 25%, oder bevorzugt um 30% oder bevorzugt um 40% verringert, bezogen auf die Netzbogenlänge des Polymers vor dem Druckprozess.

Die Glasübergangstemperatur, $T_g$, steigt bevorzugt über den 2-stufigen Prozess um 5 °C, oder bevorzugt um 7 °C, oder bevorzugt um 8 °C, oder bevorzugt um 9 °C, bezogen auf $T_g$ des Polymers vor dem Druckprozess.

Der Modul steigt bevorzugt über den 2-stufigen Prozess bevorzugt um 20%, oder bevorzugt um 30%, oder bevorzugt um 50%, oder bevorzugt um 100%, bezogen auf den Modul des Polymers vor dem Druckprozess.

Die Bruchfestigkeit kann über den 2-stufigen Prozess bevorzugt um ≥ 15%, oder bevorzugt um ≥ 20%, um ≥ 25% und weiterhin bevorzugt um ≥ 30%, bezogen auf die Bruchfestigkeit des Polymers vor dem Druckprozess steigen.

Beispiele

Die vorliegende Erfindung wird anhand der nachfolgenden Beispiele näher erläutert, ohne jedoch darauf beschränkt zu sein. Erfindungsgemäße Versuche in Tabellen 1 und 2 sind mit einem Stern (*) gekennzeichnet.

DMA-Messungen wurden in Anlehnung an die Norm DIN EN ISO 6721 durchgeführt. Ein Probekörper mit bekannter Geometrie wurde einer mechanischen nicht resonanten Schwingung in Zug bei einer konstanter Frequenz von 1 Hz und einer Temperatur von 0 °C bis 80 °C in einem Gerät vom Typ Mettler Toledo DMA 861 unterworfen. Wie in der Norm beschrieben wurde aus den Kraft- und Deformations-Messwerten und der Phasenverschiebung zwischen Kraft und Verformungssignal der Zugspeichermodul (E‘) und Zugverlustmodul (E") berechnet. Die Prüfanordnung entsprach Teil 4 der ISO 6721.

**Beispiel 1: Herstellung von Urethanacrylat 1 aus Desmodur® N3600 und Hydroxypropylacrylat.**

In einem Glaskolben wurden 100 g des trifunktionellen Isocyanatvernetzers Desmodur ® N 3600 (HDI-Trimer; bezogen von der Covestro Deutschland AG, Deutschland) bei Raumtemperatur vorgelegt. Zum Isocyanat wurden zunächst 0,040 g Dibutylzinnlaurat hinzugegeben und anschließend wurden äquimolar Hydroxypropylacrylat bezogen von Sigma-Aldrich, Deutschland über einen Zeitraum von ca. 30 Minuten hinzugetropft. Anschließend wurde die Reaktionsmischung auf 60 °C mittels temperiertem Ölbad solange erhitzt, bis der theoretische Rest-NCO-Gehalt von 0 % erreicht wurde. Dazu wurden in regelmäßigen Abständen Proben aus dem Reaktionsgefäß gezogen und titrimetrisch nach DIN EN ISO 11909 bestimmt.

Nach Erreichen des theoretischen Rest-NCO-Gehalts wurden 0,20 g des Inhibitors Butylhydroxytoluol hinzugegeben und die Mischung für 15 Minuten homogenisiert. Nach Abkühlen auf 50 °C wurde dann das erhaltene Reaktionsgemisch auf 80% mit Hexamethylendioldiacrylat (HDDA) verdünnt.

**Beispiel 2: Herstellung von Urethanacrylat 2, eines Prepolymers mit blockierten Isocyanaten und Acrylatfunktionen.**

In einem Glaskolben wurden 130,0 g des linearen Polypropylenetherpolyols Desmophen® 1111BD (bezogen

von der Covestro Deutschland AG, Deutschland) bei Raumtemperatur vorgelegt. Zum Polyol wurden zunächst 0,043 g Dibutylzinnlaurat hinzugegeben und anschließend wurden 101,9 g des Hexamethylendiisocyanat-basierten Uretdions Desmodur® N 3400 (bezogen von der Covestro Deutschland AG, Deutschland) über einen Zeitraum von ca. 30 Minuten hinzugetropft. Anschließend wurde die Reaktionsmischung auf 80 °C mittels temperiertem Ölbad solange erhitzt, bis der theoretische Rest-NCO-Gehalt von 4,71 % erreicht wurde. Dazu wurden in regelmäßigen Abständen Proben aus dem Reaktionsgefäß gezogen und titrimetrisch nach DIN EN ISO 11909 bestimmt.

Nach Erreichen des theoretischen Rest-NCO-Gehalts wurden 0,20 g des Inhibitors Butylhydroxytoluol hinzugegeben und die Mischung für 15 Minuten homogenisiert. Nach Abkühlen auf 50 °C wurden dann 33,8 g Hydroxyethylmethacrylat hinzugetropft und die Mischung so lange weitergerührt, bis ein Rest-NCO-Gehalt von 0 % erreicht wurde. Das erhaltene Reaktionsgemisch wurde auf 65% mit Isobornylmethacrylat (IBOMA) verdünnt.

**Beispiel 3: Herstellung des radikalisch vernetzbaren Harzes**

In einem Kunststoffbecher mit Deckel wurden entsprechend Gewichtsanteilen aus Tabelle 1 und 2 das Urethanacrylat, der Photoinitiator und gegebenenfalls Inhibitor eingewogen. Diese Einsatzstoffe wurden in einem Planetenkreiselmischer Thinky ARE250 bei Raumtemperatur für ca. 2 Minuten bei einer Umdrehungszahl von 2000 Umdrehungen pro Minute vermischt. Anschließend wurden die in Tabelle 1 und 2 angegebenen Mengen von n-Butylacrylat und/oder Isobornylacrylat (IBOA) hinzugegeben und mit einem Spatel manuell vermischt.

Gegebenenfalls wurde in einem weiteren Schritt Butandiol auf 40 °C erwärmt und hinzugegeben und mit einem Spatel manuell vermischt.

**Beispiel 4: Härtung des radikalisch vernetzbaren Harzes**

Das radikalisch vernetzbare Harz wurde mit Rakeln unterschiedlichen Spaltmaßes übereinander auf eine Glasplatte aufgezogen. Hierdurch wurde ein 3D-Druckverfahren im Sinne eines DLP 3D-Druckers simuliert. Die Glasplatte wurde vorab mit einer 1%-igen Lösung von Sojalecithin in Ethylacetat behandelt und getrocknet. Das Sojalecithin wirkte als Trennmittel, um später die gehärteten Filme wieder von Substrat lösen zu können. Die Spaltmaße betrugen 300 μm, 200 μm und 100 μm.

Die jeweiligen aufgetragenen Schichten wurden jeweils in einer UV-Härtungsanlage der Firma *Superfici* mit Quecksilber- und Gallium-Strahlungsquellen mit einer Bandgeschwindigkeit von 5 m/min gehärtet. Aus Lampenleistung und Bandgeschwindigkeit resultierte eine Strahlungsintensität von 1300 $mJ/cm^2$, die auf die beschichteten Substrate einwirkte. Dadurch entstand ein Drei-Schichtaufbau von insgesamt ca. 600 μm Dicke. Die Proben wurden im Anschluss an die Härtung für 12 Stunden bei 60 °C im Umluftofen temperiert.

Die gehärteten Filme wurden vorsichtig von den Glassubstraten abgezogen, um Probekörper für die mechanische Charakterisierung zu erhalten. Es wurden weiterhin haptische und optische Beurteilungen der gehärteten Filme durchgeführt.

Tabelle 1: Rezepturen UV-härtbarer Harzmischungen mit Urethanacrylat 1 (Isocyanurat enthaltend). Die Mengen sind in Gewichtsteilen angegeben.

| Versuch Nr. | 1a | 1b | 1c | 1d | 1e* | 1* | 2 | 3 | 4 | 5 | 6 | 7 | 8 |
|---|---|---|---|---|---|---|---|---|---|---|---|---|---|
| Urethanacrylat 1 (einschließlich HDDA) | 20 | 21 | 21 | 20 | 20 | 21 | 20 | 20 | 20 | 20 | 20 | 20 | 21 |
| | | | | | | | | | | | | | |
| IBOA | 80 | 75 | 70 | 65 | 60 | 55 | 50 | 45 | 40 | 35 | 30 | 25 | 20 |
| | | | | | | | | | | | | | |
| n-Butylacrylat | 0 | 5 | 10 | 15 | 20 | 25 | 30 | 35 | 40 | 45 | 50 | 55 | 60 |
| | | | | | | | | | | | | | |
| Photoinitiator | 5 | 5 | 5 | 5 | 5 | 5 | 5 | 5 | 5 | 5 | 5 | 5 | 5 |
| | | | | | | | | | | | | | |
| Haptik bei 35 °C | Hart, spröde | Hart, spröde | Hart, leicht spröde | Hart | Hart, zäh | Hart, zäh | Zäh | Zäh | Weich | Weich | Kontaktklebrig | Kontaktklebrig | Kontaktklebrig |
| | | | | | | | | | | | | | |
| $T_g$ (tan $\delta$)/DMA [°C] | | 78 | 69 | | | 47 | | | | | | | |
| E' 35 °C/DMA [MPa] | | 2095 | 2023 | | | 1456 | | | | | | | |
| tan $\delta$ 35 °C/DMA | | 0,037 | 0,040 | | | 0,254 | | | | | | | |

Alle Proben waren klar und wiesen eine hohe Transparenz auf. FIG. 1 zeigt DMA-Kurven einer Probe aus dem erfindungsgemäßen Versuch Nr. 1. FIG. 2 zeigt DMA-Kurven aus dem Versuch Nr. 1b (Vergleichsbeispiel). FIG. 3 zeigt DMA-Kurven aus dem Versuch Nr. 1c (Vergleichsbeispiel).

Tabelle 2: Rezepturen UV-härtbarer Harzmischungen mit Urethanacrylat 2 (Uretdion enthaltend). Die Mengen sind in Gewichtsteilen angegeben.

| Versuch Nr. | **9a** | **9b** | **9c** | **9e** | **9f*** | **9*** | **10** | **11** | **12** | **13** | **14** | **15** | **16*** |
|---|---|---|---|---|---|---|---|---|---|---|---|---|---|
| Urethanacrylat 2 (einschließlich IBOMA) | 21 | 22 | 21 | 20 | 20 | 21 | 20 | 21 | 20 | 20 | 20 | 20 | 20 |
| | | | | | | | | | | | | | |
| IBOA | 80 | 75 | 70 | 63 | 60 | 55 | 50 | 45 | 40 | 35 | 30 | 25 | 65 |
| | | | | | | | | | | | | | |
| n-Butylacrylat | 0 | 5 | 10 | 17 | 20 | 25 | 30 | 35 | 40 | 45 | 50 | 55 | 15 |
| | | | | | | | | | | | | | |
| Butandiol | 0 | 0 | 0 | 0 | 0 | 0 | 0 | 0 | 0 | 0 | 0 | 0 | 5 |
| Photoinitiator | 0 | 5 | 5 | 5 | 5 | 5 | 5 | 5 | 5 | 5 | 5 | 5 | 5 |
| | | | | | | | | | | | | | |
| Haptik bei 40 °C | Hart, spröde | Hart, spröde | Hart, leicht spröde | Hart, zäh | Hart, zäh | Hart, zäh | Zäh | Weich | Weich | Kontaktklebrig | Kontaktklebrig | Kontaktklebrig | Hart, zäh, klebrig |
| | | | | | | | | | | | | | |
| $T_g$ (tan $\delta$)/DMA [°C] | | | 71 | | | 38 | | | | | | | |
| E' 35 °C/DMA [MPa] | | | 2041 | | | 534 | | | | | | | |
| tan $\delta$ 35 °C/DMA | | | 0,05 | | | 0,136 | | | | | | | |

Alle Proben waren klar und wiesen eine hohe Transparenz auf. FIG. 4 zeigt DMA-Kurven aus dem erfindungsgemäßen Versuch Nr. 9. FIG. 5 zeigt DMA-Kurven aus dem Versuch Nr. 9c (Vergleichsbeispiel).

## Patentansprüche

1. Kieferorthopädische Zahnschiene,

   **dadurch gekennzeichnet, dass**
   die Zahnschiene ein vernetztes Polymer umfasst oder aus einem solchen besteht, wobei das vernetzte Polymer eine Glasübergangstemperatur $T_g$, bestimmt mittels dynamisch-mechanischer Analyse bei einer Frequenz von 1/s als Peak tan $\delta$, von $\geq$ 25 °C und $\leq$ 60 °C, ein Elastizitätsmodul, bestimmt mittels dynamisch-mechanischer Analyse als Speichermodul E' bei einer Frequenz von 1/s bei 35 °C, von $\geq$ 500 MPa und $\leq$ 4000 MPa und einen Verlustfaktor tan $\delta$, bestimmt mittels dynamisch-mechanischer Analyse bei einer Frequenz von 1/s bei 35 °C, von $\geq$ 0,08 aufweist und
   wobei das vernetzte Polymer ein Copolymer ist, welches Einheiten basierend auf einem ersten Monomer und einem zweiten Monomer enthält, wobei das erste Monomer ein (meth)acrylisches Monomer ist, dessen Homopolymer eine Glasübergangstemperatur, bestimmt mittels dynamisch-mechanischer Analyse bei einer Frequenz von 1/s als Peak tan $\delta$, von $\leq$ 0 °C aufweist und das zweite Monomer ein (meth)acrylisches oder styrolisches Monomer ist, dessen Homopolymer eine Glasübergangstemperatur, bestimmt mittels dynamisch-mechanischer Analyse bei einer Frequenz von 1/s als Peak tan $\delta$, von $\geq$ 60 °C aufweist und wobei Einheiten basierend auf dem ersten Monomer in einem Anteil von $\geq$ 5 bis $\leq$ 40 Gewichts-%, bezogen auf das Gesamtgewicht des vernetzten Polymers, vorliegen und Einheiten basierend auf dem zweiten Monomer in einem Anteil von $\geq$ 20 bis $\leq$ 80 Gewichts-%, bezogen auf das Gesamtgewicht des vernetzten Polymers, vorliegen.

2. Zahnschiene gemäß Anspruch 1, wobei das vernetzte Polymer ein vernetztes Polyurethan umfasst.

3. Zahnschiene gemäß einem der vorhergehenden Ansprüche, wobei das vernetzte Polymer einen Isocyanuratanteil, ermittelt über $^{13}$C-NMR, von $\geq$ 3% aufweist.

4. Zahnschiene gemäß Anspruch 2 oder 3, wobei das vernetzte Polymer einen Urethananteil ermittelt über $^{13}$C NMR von $\geq$ 3% aufweist.

5. Zahnschiene gemäß einem der vorhergehenden Ansprüche, wobei das vernetzte Polymer einen Brechungsindex gemessen mit einem Abbe Refraktometer von >1,48 RI und < 1,58 RI aufweist.

6. Zahnschiene gemäß einem der vorhergehenden Ansprüche, wobei das vernetzte Polymer eine mittlere Netzbogenlänge nach Flory und Huggins von > 300 g/mol und < 5000 g/mol aufweist.

7. Zahnschiene nach einem der vorhergehenden Ansprüche, wobei das Polymer ein transparentes Polymer mit einer Lichttransmission gemessen in einem UV-VIS Spektrometer an einer Probe mit einer Dicke von 1 mm im Wellenlängenbereich von 400-800 nm von >50% ist.

8. Zahnschiene nach einem der vorhergehenden Ansprüche, wobei das Polymer ein transparentes Polymer enthaltend Polyurethane und / oder Polysilicone ist und eine Abbe Zahl von > 20 aufweist.

9. Verfahren zur Herstellung einer kieferorthopädischen Zahnschiene, umfassend die Schritte:

   i) Auswahl eines vernetzbaren Harzes;
   ii) Formen der Zahnschiene durch Vernetzen des in Schritt i) ausgewählten vernetzbaren Harzes

      unter Ausbildung eines vernetzten Polymers;
      **dadurch gekennzeichnet, dass**
      die Auswahl in Schritt i) das Kriterium umfasst, dass ein nach Vernetzung des vernetzbaren Harzes erhaltenes vernetztes Polymer eine Glasübergangstemperatur $T_g$, bestimmt mittels dynamisch-mechanischer Analyse bei einer Frequenz von 1/s DMA als Peak tan $\delta$, von $\geq$ 25 °C und $\leq$ 60 °C, ein Elastizitätsmodul, bestimmt mittels dynamisch-mechanischer Analyse als Speichermodul E' bei einer Frequenz von 1/s bei 35 °C, von $\geq$ 500 MPa und $\leq$ 4000 MPa und einen Verlustfaktor tan $\delta$, bestimmt mittels dynamisch-mecha-

nischer Analyse bei einer Frequenz von 1/s bei 35 °C, von ≥ 0,08 aufweist und
wobei das vernetzbare Harz ein erstes Monomer und ein zweites Monomer enthält, wobei das erste Monomer ein (meth)acrylisches Monomer ist, dessen Homopolymer eine Glasübergangstemperatur, bestimmt mittels dynamisch-mechanischer Analyse bei einer Frequenz von 1/s als Peak tan δ, von ≤ 0 °C aufweist und das zweite Monomer ein (meth)acrylisches oder styrolisches Monomer ist, dessen Homopolymer eine Glasübergangstemperatur, bestimmt mittels dynamisch-mechanischer Analyse bei einer Frequenz von 1/s als Peak tan δ, von ≥ 60 °C aufweist und wobei das erste Monomers in einem Anteil von ≥ 5 bis ≤ 40 Gewichts-%, bezogen auf das Gesamtgewicht des Harzes, vorliegt und das zweite Monomer in einem Anteil von ≥ 20 bis ≤ 80 Gewichts-%, bezogen auf das Gesamtgewicht des Harzes, vorliegen.

**10.** Verfahren nach Anspruch 9, wobei in Schritt ii) die Zahnschiene durch Vernetzen des vernetzbaren Harzes in einer zu der Zahnschiene korrespondierenden Gießform geformt wird.

**11.** Verfahren nach Anspruch 9, wobei in Schritt ii) die Zahnschiene mittels eines additiven Herstellungsverfahrens geformt wird.

**12.** Verfahren nach einem der Ansprüche 9 oder 10, wobei das vernetzbare Harz freie Isocyanatgruppen gemessen mittels $^{13}C$ NMR in einer Konzentration ≥ 1 Gew.-% bezogen auf das vernetzbare Harz aufweist.

**13.** Verfahren nach einem der Ansprüche 9 bis 12, wobei das vernetzbare Harz freie Alkoholgruppen gemessen mittels $^{13}C$ NMR in einer Konzentration ≥ 0,5 Gew.-% bezogen auf das vernetzbare Harz aufweist.

**Claims**

**1.** An orthodontic aligner,

**characterized in that**
the aligner comprises a crosslinked polymer or consists of such a polymer, said crosslinked polymer having a glass transition temperature $T_g$, determined by dynamic mechanical analysis at a frequency of 1/s as peak tan δ, of ≥ 25°C and ≤ 60°C, an elasticity modulus, determined by dynamic mechanical analysis as storage modulus E' at a frequency of 1/s at 35°C, of ≥ 500 MPa and ≤ 4000 MPa and a loss factor tan δ, determined by dynamic mechanical analysis at a frequency of 1/s at 35°C, of ≥ 0.08 and
wherein the crosslinked polymer is a copolymer, which comprises units based on a first monomer and a second monomer, said first monomer being a (meth)acrylic monomer whose homopolymer has a glass transition temperature, determined by dynamic mechanical analysis at a frequency of 1/s as peak tan δ, of ≤ 0°C, and said second monomer being a (meth)acrylic or styrenic monomer whose homopolymer has a glass transition temperature, determined by dynamic mechanical analysis at a frequency of 1/s as peak tan δ, of ≥ 60°C, and where units based on the first monomer are present in a fraction of ≥ 5 to ≤ 40 weight%, based on the total weight of the crosslinked polymer, and units based on the second monomer are present in a fraction of ≥ 20 to ≤ 80 weight%, based on the total weight of the crosslinked polymer.

**2.** The aligner as claimed in claim 1, wherein the crosslinked polymer comprises a crosslinked polyurethane.

**3.** The aligner as claimed in any of the preceding claims, wherein the crosslinked polymer has an isocyanurate fraction, ascertained via $^{13}C$ NMR, of ≥ 3%.

**4.** The aligner as claimed in claim 2 or 3, wherein the crosslinked polymer has a urethane fraction, ascertained via $^{13}C$ NMR, of ≥ 3%.

**5.** The aligner as claimed in any of the preceding claims, wherein the crosslinked polymer has a refractive index, measured with an Abbe refractometer, of ≥ 1.48 RI and < 1.58 RI.

**6.** The aligner as claimed in any of the preceding claims, wherein the crosslinked polymer has a mean network arc length according to Flory and Huggins of ≥ 300 g/mol and < 5000 g/mol.

**7.** The aligner as claimed in any of the preceding claims, wherein the polymer is a transparent polymer having a light transmittance, measured in a UV-VIS spectrometer on a sample with a thickness of 1 mm in the wavelength range

of 400-800 nm, of ≥ 50%.

**8.** The aligner as claimed in any of the preceding claims, wherein the polymer is a transparent polymer comprising polyurethanes and/or polysilicones and has an Abbe number of ≥ 20.

**9.** A method for producing an orthodontic aligner, comprising the steps of:

i) selecting a crosslinkable resin;
ii) shaping the aligner by crosslinking the crosslinkable resin selected in step i), to form a crosslinked polymer;

**characterized in that**
the selection in step i) includes the criterion that a crosslinked polymer obtained after crosslinking of the crosslinkable resin has a glass transition temperature $T_g$, determined by dynamic mechanical analysis at a frequency of 1/s DMA as peak tan δ, of ≥ 25°C and ≤ 60°C, an elasticity modulus, determined by dynamic mechanical analysis as storage modulus E' at a frequency of 1/s at 35°C, of ≥ 500 MPa and ≤ 4000 MPa and a loss factor tan δ, determined by dynamic mechanical analysis at a frequency of 1/s at 35°C, of ≥ 0.08 and wherein the crosslinkable resin comprises a first monomer and a second monomer, said first monomer being a (meth)acrylic monomer whose homopolymer has a glass transition temperature, determined by dynamic mechanical analysis at a frequency of 1/s as peak tan δ, of ≤ 0°C, and said second monomer being a (meth)acrylic or styrenic monomer whose homopolymer has a glass transition temperature, determined by dynamic mechanical analysis at a frequency of 1/s as peak tan δ,, of ≥ 60°C, and where the first monomer is present in a fraction of ≥ 5 to ≤ 40 weight%, based on the total weight of the resin, and the second monomer is present in a fraction of ≥ 20 to ≤ 80 weight%, based on the total weight of the resin.

**10.** The method as claimed in claim 9, wherein the aligner is shaped in step ii) by crosslinking the crosslinkable resin in a casting mold corresponding to the aligner.

**11.** The method as claimed in claim 9, wherein the aligner is shaped in step ii) by means of an additive manufacturing method.

**12.** The method as claimed in any of claims 9 to 10, wherein the crosslinkable resin has free isocyanate groups, measured by $^{13}C$ NMR, in a concentration ≥ 1 wt%, based on the crosslinkable resin.

**13.** The method as claimed in any of claims 9 to 12, wherein the crosslinkable resin has free alcohol groups, measured by $^{13}C$ NMR, in a concentration ≥ 0.5 wt%, based on the crosslinkable resin.

## Revendications

**1.** Gouttière dentaire orthodontique,

**caractérisée en ce que**
la gouttière dentaire comprend un polymère réticulé ou est constituée de celui-ci, le polymère réticulé ayant une température de transition vitreuse $T_g$, déterminée par analyse dynamico-mécanique à une fréquence de 1/s comme pic tan δ, de ≥ 25 °C et ≤ 60 °C, un module d'élasticité déterminé par analyse dynamico-mécanique comme module de stockage E' à une fréquence de 1/s à 35 °C, de ≥ 500 MPa et ≤ 4000 MPa et un facteur de perte tan δ déterminé par analyse dynamico-mécanique à une fréquence de 1/s à 35 °C, de ≥ 0,08 et
le polymère réticulé étant un copolymère qui contient des motifs basé sur un premier monomère et un deuxième monomère, le premier monomère étant un monomère (méth)acrylique dont l'homopolymère a une température de transition vitreuse, déterminée par analyse dynamico-mécanique à une fréquence de 1/s comme pic tan δ, de ≤ 0 °C et le deuxième monomère étant un monomère (méth)acrylique ou styrénique dont l'homopolymère a une température de transition vitreuse, déterminée par analyse dynamico-mécanique à une fréquence de 1/s comme pic tan δ, de ≥ 60 °C et des motifs basés sur le premier monomère étant présents dans une proportion de ≥ 5 à ≤ 40 % en poids par rapport au poids total du polymère réticulé, et des motifs à base du second monomère étant présents dans une proportion ≥ 20 à ≤ 80 % en poids par rapport au poids total du polymère réticulé.

**2.** Gouttière dentaire selon la revendication 1, le polymère réticulé comprenant un polyuréthane réticulé.

**3.** Gouttière dentaire selon l'une des revendications précédentes, le polymère réticulé ayant une teneur en isocyanurate, déterminée par $^{13}C$-NMR, de ≥ 3 %.

**4.** Gouttière dentaire selon la revendication 2 ou 3, le polymère réticulé ayant une teneur en uréthane déterminée par $^{13}C$-NMR, de ≥ 3 %.

**5.** Gouttière dentaire selon l'une des revendications précédentes, le polymère réticulé ayant un indice de réfraction, mesuré au réfractomètre d'Abbe, de > 1,48 RI et < 1,58 RI.

**6.** Gouttière dentaire selon l'une des revendications précédentes, le polymère réticulé ayant une longueur moyenne d'arc de réseau selon Flory et Huggins de > 300 g/ mol et < 5000 g/mol.

**7.** Gouttière dentaire selon l'une des revendications précédentes, le polymère étant un polymère transparent avec une transmission lumineuse de > 50 %, mesurée dans un spectromètre UV-VIS sur un échantillon d'une épaisseur de 1 mm dans la gamme de longueur d'onde de 400 à 800 nm.

**8.** Gouttière dentaire selon l'une des revendications précédentes, le polymère étant un polymère transparent qui contient des polyuréthanes et/ou des polysilicones et ayant un nombre d'Abbe > 20.

**9.** Procédé de fabrication d'une gouttière dentaire orthodontique, ledit procédé comprenant les étapes suivantes :

i) sélectionner une résine réticulable ;
ii) mettre en forme la gouttière dentaire par réticulation de la résine réticulable sélectionnée à l'étape i) pour former un polymère réticulé ; **caractérisé en ce que**

la sélection à l'étape i) comprend le critère selon lequel un polymère réticulé obtenu après réticulation de la résine réticulable a une température de transition vitreuse $T_g$, déterminée par analyse dynamico-mécanique DMA à une fréquence de 1/s comme pic tan $\delta$, de ≥ 25 °C et ≤ 60 °C, un module d'élasticité, déterminé par analyse dynamico-mécanique comme module de stockage E' à une fréquence de 1/s à 35 °C, de ≥ 500 MPa et ≤ 4000 MPa et un facteur de perte tan $\delta$, déterminé par analyse dynamico-mécanique à une fréquence de 1/s à 35 °C, de ≥ 0,08 et
la résine réticulable contenant un premier monomère et un deuxième monomère, le premier monomère étant un monomère (méth)acrylique dont l'homopolymère a une température de transition vitreuse, déterminée par analyse dynamico-mécanique à une fréquence de 1/s comme pic tan $\delta$, de ≤ 0 °C et le deuxième monomère étant un monomère (méth)acrylique ou styrénique dont l'homopolymère a une température de transition vitreuse, déterminée par analyse dynamico-mécanique à une fréquence de 1/s comme pic tan $\delta$, de ≥ 60 °C et le premier monomère étant présent dans une proportion de ≥ 5 à ≤ 40 % en poids, par rapport au poids total de la résine et le deuxième monomère étant présent dans une proportion de ≥ 20 à ≤ 80 % en poids sur la base du poids total de la résine.

**10.** Procédé selon la revendication 9, à l'étape ii) la gouttière dentaire étant formée par réticulation de la résine réticulable dans un moule correspondant à la gouttière dentaire.

**11.** Procédé selon la revendication 9, à l'étape ii) la gouttière dentaire étant formée au moyen d'un procédé de fabrication additive.

**12.** Procédé selon l'une des revendications 9 et 10, la résine réticulable comportant des groupements isocyanates libres mesurés par $^{13}C$-NMR à une concentration ≥ 1 % en poids par rapport à la résine réticulable.

**13.** Procédé selon l'une des revendications 9 à 12, la résine réticulable comportant des groupements alcool libres mesurés par $^{13}C$-NMR à une concentration ≥ 0,5 % en poids par rapport à la résine réticulable.

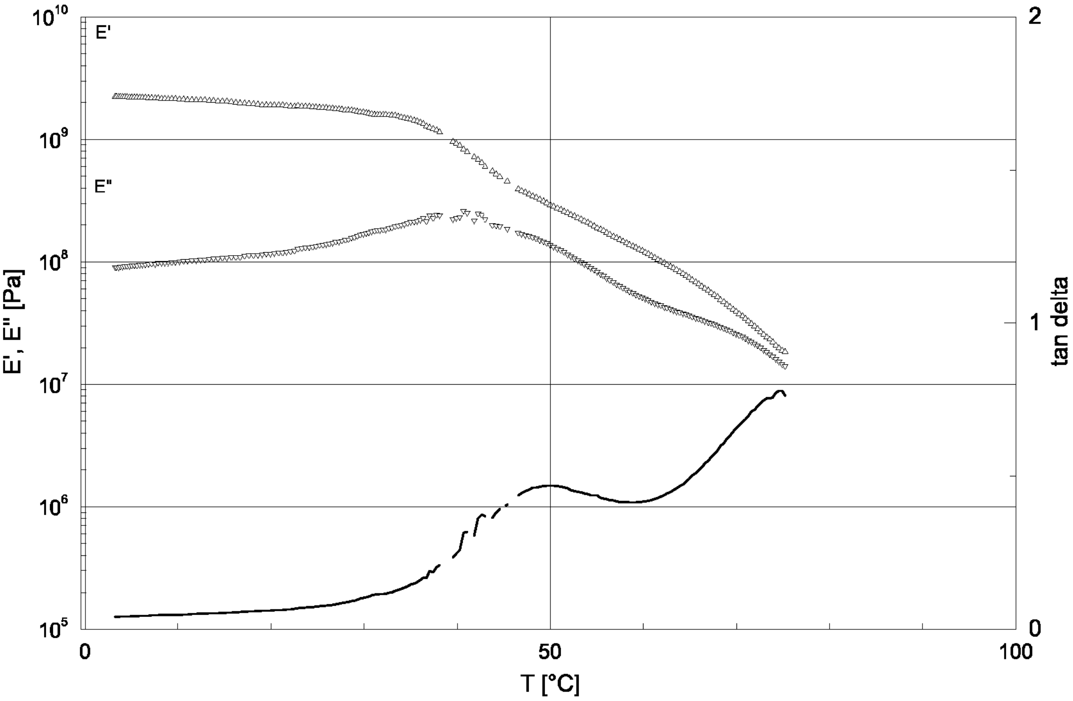
E'
E"
E', E" [Pa]
tan delta
T [°C]
$10^{10}$
$10^{9}$
$10^{8}$
$10^{7}$
$10^{6}$
$10^{5}$
0
50
100
2
1
0

FIG. 1

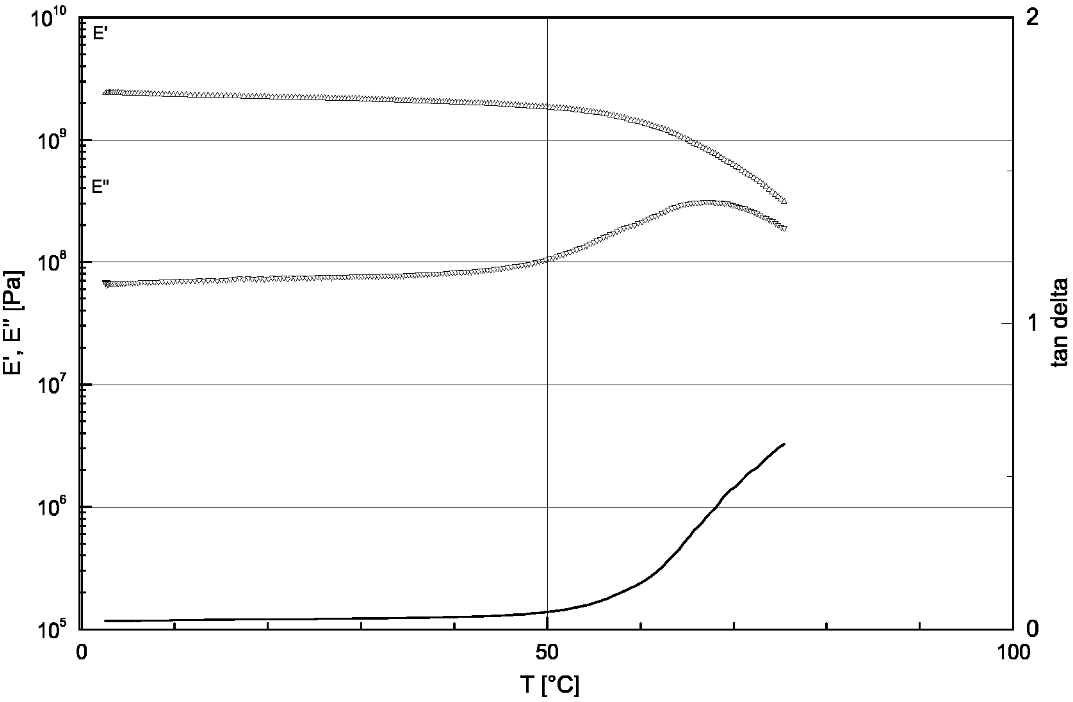
E'
E"
E', E" [Pa]
tan delta
T [°C]
$10^{10}$
$10^{9}$
$10^{8}$
$10^{7}$
$10^{6}$
$10^{5}$
0
50
100
2
1
0

FIG. 2

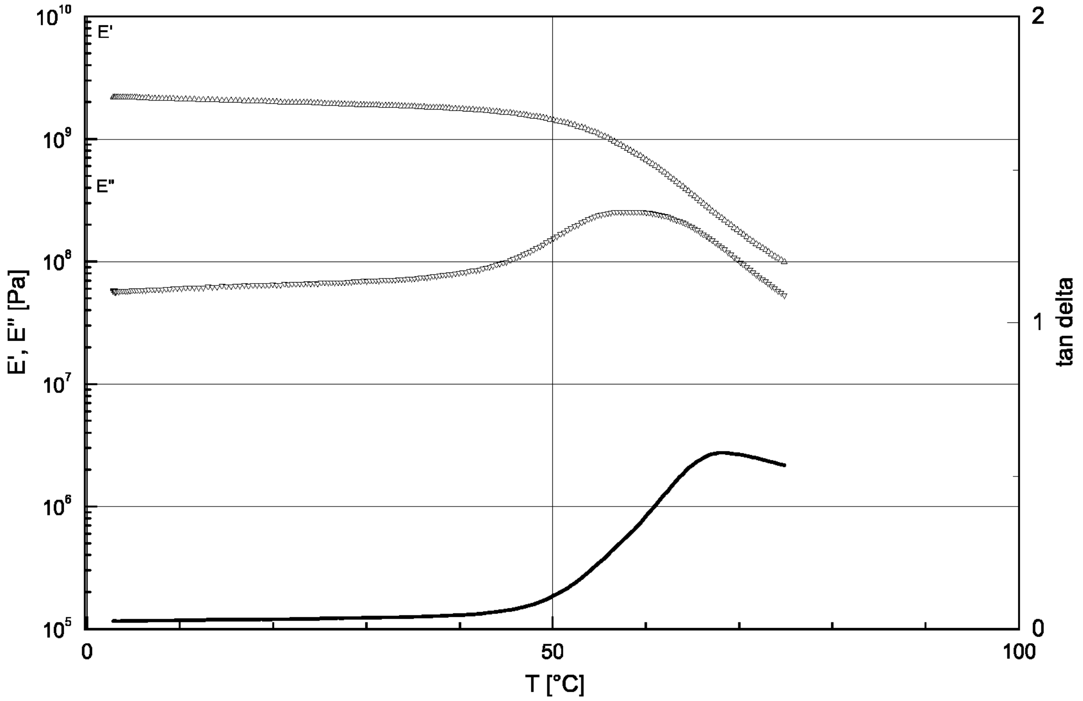
E'
E"
E', E" [Pa]
tan delta
T [°C]
$10^{10}$
$10^{9}$
$10^{8}$
$10^{7}$
$10^{6}$
$10^{5}$
0
50
100
2
1
0

FIG. 3

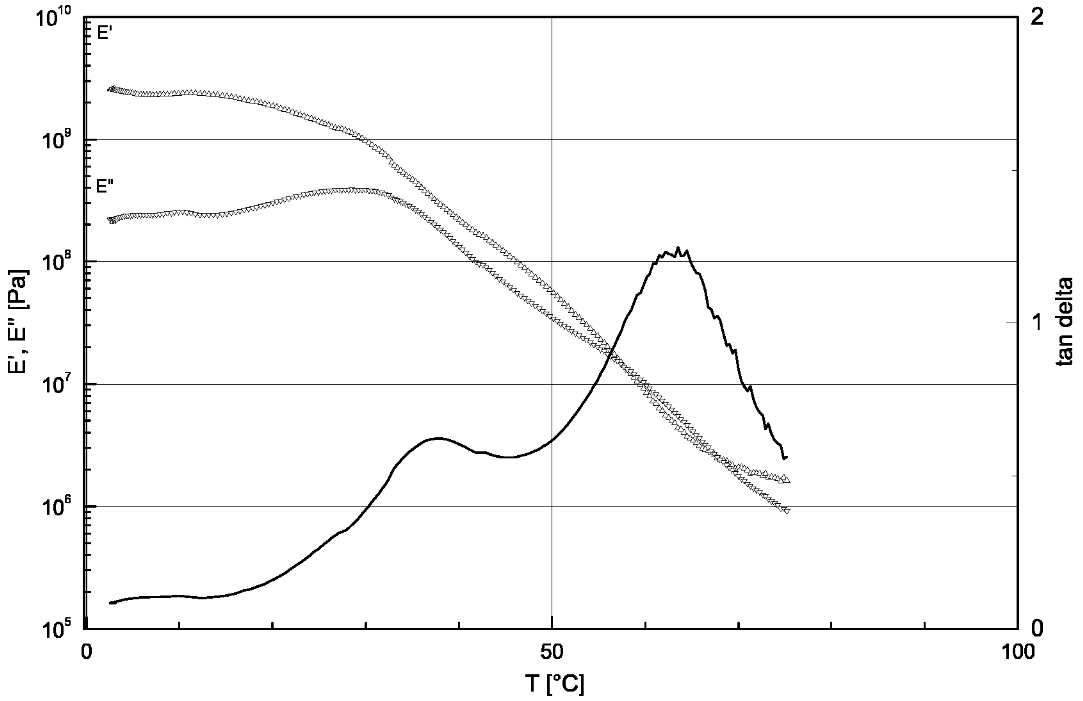
E'
E"
E', E" [Pa]
tan delta
T [°C]
$10^{10}$
$10^{9}$
$10^{8}$
$10^{7}$
$10^{6}$
$10^{5}$
0
50
100
2
1
0

FIG. 4

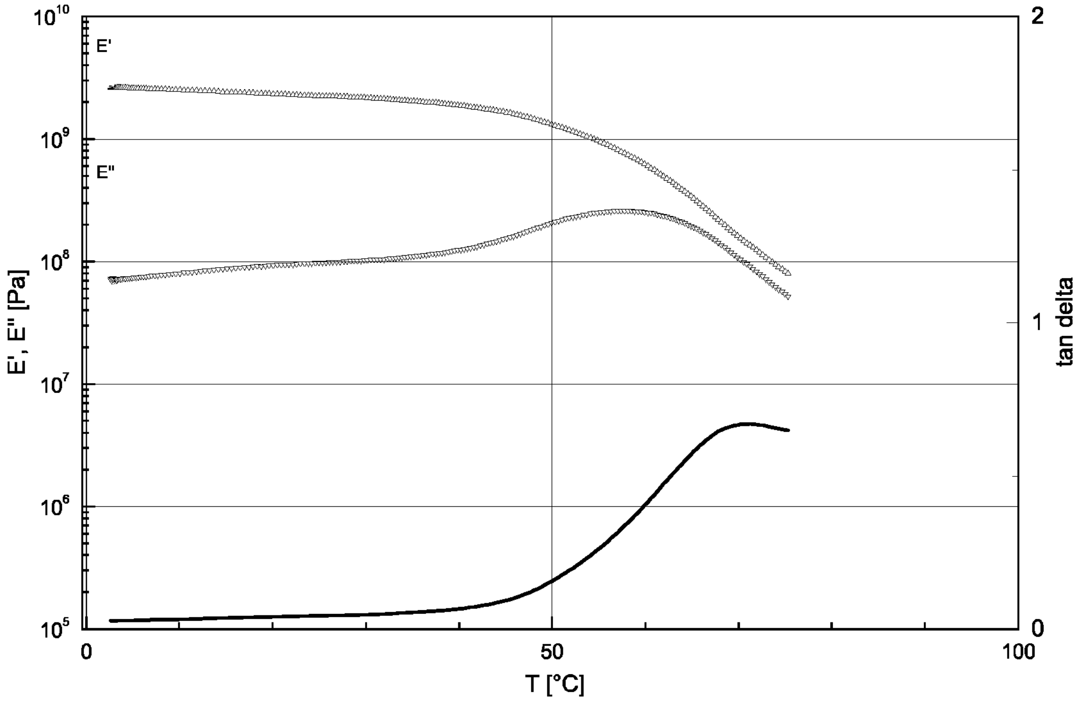


FIG. 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20160256240 A1
- US 20130095446 A1
- US 20130122448 A1
- US 5975893 A
- WO 2018005501 A1